# EUROPEAN PATENT SPECIFICATION

(11) **EP 3 715 321 B1**
(45) Date of publication and mention of the grant of the patent: **26.11.2025**
(21) Application number: 20162294.1
(22) Date of filing: 11.03.2020
(51) Int. Cl.: C01B 21/086, C01B 21/093, H01M 10/0525, H01M 10/0568

(54) **PROCESS FOR THE PREPARATION OF A BISFLUOROSULFONYLIMIDE SALT**
VERFAHREN ZUR HERSTELLUNG VON EINEM BISFLUORSULFONYLIMIDSALZ
PROCÉDÉ DE PRÉPARATION D'UN SEL DE BISFLUOROSULFONYLIMIDE

(30) Priority: 11.03.2019 CN 201910178675
(43) Date of publication of application: 30.09.2020
(73) Proprietor: Shanghai Rolechem Co., Ltd., Shanghai 201207 (CN)
(72) Inventor: Shen, Fengfeng, Shanghai, 201207 (CN)
(74) Representative: Canzler & Bergmeier Patentanwälte Partnerschaft mbB

(56) References cited:
- EP-A1- 3 466 871
- WO-A1-2014/035464
- WO-A1-2017/204225
- CN-B- 104 495 767
- MARTIN BERAN ET AL: "A New Method of the Preparation of Imido-bis(sulfuric acid) Dihalogenide, (F, Cl), and the Potassium Salt of Imido-bis(sulfuric acid) Difluoride", ZEITSCHRIFT FUR ANORGANISCHE UND ALLGEMEINE CHEMIE, vol. 631, no. 1, 1 January 2005 (2005-01-01), pages 55 - 59, XP055014688, ISSN: 0044-2313, DOI: 10.1002/zaac.200400325

## Description

### FIELD OF THE INVENTION

The present disclosure relates to a method for preparing abisfluorosulfonylimide salt, and more particularly relates to a new process for abisfluorosulfonylimide salt.

### BACKGROUND OF THE INVENTION

Document WO 2017/204 225 A1 discloses a method for producing a bis(fluorosulfonyl)imide alkali metal salt by causing a reaction of a mixture that contains bis(fluorosulfonyl)imide and an alkali metal compound. According to this method for producing a bis(fluorosulfonyl)imide alkali metal salt, the total of the weight ratios of the bis(fluorosulfonyl)imide, the alkali metal compound and the bis(fluorosulfonyl)imide alkali metal salt to the entire mixture after the reaction is 0.8 or more.

Document CN 104 495 767B discloses a method for preparing lithium bisfluorosulfonimide including the steps: A) reacting sulfuryl chloride and ammonia gas are reacted in the presence of an organic base to obtain an organic base salt of bischlorosulfonimide; B) mixing tThe organic base salt of bischlorosulfonimide obtained in step A) with HF, and performing the fluorination reaction to obtain the organic base salt of bisfluorosulfonimide; C) mixing the bischlorosulfonimide obtained in step B), mixing the organic alkali salt of fluorosulfonimide with the alkaline substance, and carrying out the neutralization reaction to obtain the crude bisfluorosulfonimide alkali metal salt; D) purifying the bisfluorosulfonimide alkali metal obtained in step C) from the crude salt to obtain the bisfluorosulfonimide alkali metal salt; E) mixing the bisfluorosulfonimide alkali metal salt obtained in the step D) with the lithiation reagent, and performing the displacement reaction to obtain the bisfluorosulfonyl Lithium imide.

Bisfluorosulfonylimide salts (hereinafter abbreviated as MFSI) have been widely used in the field of electrolytes. For example, potassiumbisfluorosulfonylimide (also known as KFSI) is used as an additive to super capacitors,and bisfluorosulfonylimide salts are used as electrolytes (also known as LiFSI): USpatent US5916475 discloses a fluorine-containing lithium salt: lithiumbisfluorosulfonylimide (LiFSI), which has better thermal stability and chemical stability, higher conductivity and lower corrosion rate than LiTFSI and LiPF6. NIPPONSHOKUBAI Co., Ltd. achieved industrial production in 2013. At present, Japanese and South Korean companies have mixed LiPF6 with LiFSi for use at high-end occasions,and such a mixture is considered to be a type of difluorinated lithium salt that may replace LiPF6 and will have an excellent prospect of application in lithium batteries and super capacitors.

Currently, in a typical method for preparing MFSI, abischlorosulfonylimide (abbreviated as HCSI) is generally synthesized first to obtain abischlorosulfonylimide compound (HN[SO2Cl]2), (R. Appel et al, Chem. Ber. 1962, 95,625); R. Appel et al, Chem. Ber. 1962, 95, 1753; E. A. Fadia, US4315935, 1982; M.Beran et al, Z. Anorg. Allg. Chem. 2005, 631, 55), wherein M. Beran et al. proposed aone-step synthesis method that can overcome the problems of separation, toxicity and thelike, which has been widely used in recent years. In this method, HN[SO2Cl]2 is obtained by placing sulfamic acid, chlorosulfonic acid, thionyl chloride into "one pot" for reaction and vacuum-distillation. However, due to the high boiling point of the product, energy consumption in the distillation of bischlorosulfonylimide compound is relatively large.

HCSI is then reacted with hydrofluoric acid to synthesize bisfluorosulfonylimide (abbreviated as HFSI), as described in the patents CN104755418A and CN101654229A. However, a high-pressure reaction is required, which has higher requirements on the equipment. HFSI is then neutralized with a base. For example, US8377406 discloses a method for preparing LiFSI by directly reacting bisfluorosulfonylimide (HFSI) with lithium carbonate in an aqueous solution. However, HFSI decomposes after violent heat release with water. In this patent, the problem of violent heat release of HFSI when dissolved in water is solved by preparing an HFSI aqueous solution using an ultra-low temperature method (-78°C), but the energy consumption is huge. More importantly, LiFSI has very good water solubility, and it is easy to decompose under heat in the aqueous system. The extraction efficiency is very low and is not suitable for industrial production.

It has also been reported that MFSI is directly obtained by reacting HClSI with a fluoride salt. For example, US Patent US2004097757 discloses preparing LiFSI by directly reacting HClSI with LiF, but the obvious disadvantage is that a large amount of corrosive exhaust gas HF is generated, making an industrial production become difficult.

EP2894146 reported that MCSI was prepared using HCSI, and then MCSI was reacted with a fluoride salt to generate MFSI, which makes the reaction process too complicated, leading to a reduction in the total yield of the product.

Existing technologies basically all require the preparation of HFSI and HClSI, both of them are highly acidic and corrosive, which result in defects such as complicated processes, difficult product separation, high requirements on reaction equipment, difficult operation, large energy consumption and environment pollution, making it difficult to obtain LiFSI industrially.

In view of the above problems, a new process for bisfluorosulfonylimide needs to be developed in the art.

### SUMMARY OF THE INVENTION

In order to solve the above technical problems, the present disclosure provides a new process for bisfluorosulfonylimide salt.

As a preferred technical solution, a method for preparing the bisfluorosulfonylimide salt includes the following steps:
S1: reacting an N-alkyl substituted bisfluorosulfonylimide and a salt in a solvent system to obtain a crude product; and
S2: crystallizing and drying to obtain a bisfluorosulfonylimide salt.

As a preferred technical solution, a molar ratio of the N-alkyl substituted bisfluorosulfonylimide to the salt is (1: 1) to (1: 3).

As a preferred technical solution, the reaction temperature is 0°C to 150°C, and the reaction pressure is 10 KPa to 1500 KPa.

As a preferred technical solution, the structural formula of the salt is Mₙ₁Xₙ₂Oₙ₃Hₙ₄, wherein n1 is a natural number ≧ 1, and each of n2, n3 and n4 is a natural number.

As a preferred technical solution, the structural formula of the salt is Mₙ₁Xₙ₂Oₙ₃Hₙ₄; wherein n1 is 1, each of n2, n3 and n4 is 0, and the Mₙ₁Xₙ₂Oₙ₃Hₙ₄ is ammonia gas or single-element metal.

As a preferred technical solution, the structural formula of the salt is Mₙ₁Xₙ₂Oₙ₃Hₙ₄; wherein each of n1, n2, n3 and n4 is a natural number ≧ 1; the M is selected from one of ammonium, sodium, potassium, iron, calcium, lithium, copper, zinc, cadmium, chromium, aluminum, lead, nickel, molybdenum, palladium and tin; and the X is selected from one of hydrogen, boron, carbon, silicon, nitrogen, phosphorus, arsenic, oxygen, sulfur, selenium, tellurium, fluorine, chlorine, bromine, iodine, astatine, bicarbonate, carbonate, sulfate, bisulfate, nitrate, nitrite, phosphate, hydrogen phosphate, and dihydrogen phosphate.

As a preferred technical solution, the X further includes 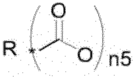 or RO, the R is -Cₙ₆Hₙ₇, wherein each of n5, n6 and n7 is a natural number ≥ 1, the R is selected from one of hydrogen, saturated aliphatic alkyl, unsaturated aliphatic alkyl, aromatic alkyl, saturated aliphatic alkyl substituted with other groups, unsaturated aliphatic alkyl substituted with other groups, aromatic alkyl substituted with other groups, and alkyl substituted with other groups; and said other groups are selected from one of formate, oxalate, hydrogen oxalate, and methoxyl.

As a preferred technical solution, the structural formula of the N-alkyl substituted bisfluorosulfonylimide is: 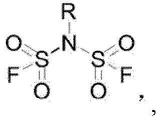 R is -Cₙ₈Hₙ₉, wherein each of n8 and n9 is a natural number ≥ 1.

As a preferred technical solution, the R is selected from one of saturated aliphatic alkyl, unsaturated aliphatic alkyl, aromatic alkyl, saturated aliphatic alkyl substituted with other elements, unsaturated aliphatic alkyl substituted with other elements, and aromatic alkyl substituted with other elements; and said other elements are selected from one of fluorine, chlorine, bromine, iodine, sulfur, phosphorus, oxygen, nitrogen, and arsenic.

As a preferred technical solution, a solvent used in the solvent system is selected from one or a combination of more than one of: diethyl carbonate, dimethyl carbonate, propylene carbonate, ethylene carbonate, ethyl methyl carbonate, methyl tert-butyl ether, tetrahydrofuran, methyl tetrahydrofuran, toluene, xylene, chlorobenzene, dichlorobenzene, trichlorobenzene, tetrachlorobenzene, methyl formate, ethyl formate, methyl acetate, ethyl acetate, propyl acetate, isopropyl acetate, n-butyl acetate, acetonitrile, ethylene glycol dimethyl ether, ethylene glycol diethyl ether, diethylene glycol dimethyl ether, acetone, diethyl ether, isopropyl ether, butyl ether, anisole, diphenyl ether, 1,4-dioxane, dichloromethane, trichloromethane, tetrachloromethane, 1,2-dichloroethane, 1,1,1-trichloroethane, 1,1,2-trichloroethane, 1,1,2,2-tetrachloroethane, 1,1,2,2-tetrachloroethylene and γ-butyrolactone.

The present disclosure has the following advantageous effects: no water is generated during the reaction, the N-alkyl substituted bisfluorosulfonylimide is stable in nature, no corrosive substances are generated during the entire reaction, and there are fewer three wastes, which makes the present disclosure suitable for large-scale production, and enables a high-purity battery-grade bisfluorosulfonylimide salt to be obtained; therefore, the present disclosure has great implementation value and social and economic benefits.

### DETAILED DESCRIPTION OF THE EMBODIMENT(S) OF THE INVENTION

The present disclosure aims to solve the technical problems that in the existing methods for preparing bisfluorosulfonylimide salt, by-products are produced, the operation is complicated, the amount of organic solvent consumption is large, the solvent is difficult to recover, and the amount of three wastes is large, which are not advantageous for an industrial production. In view of these defects, the present disclosure provides a process for synthesizing a bisfluorosulfonylimide salt with excellent performance and high purity, which includes the following operation steps: reacting RFSI with a salt to obtain a corresponding bisfluorosulfonylimide salt (M(FSON)₂); obtaining a product by reacting at a temperature of 0°C to 150°C and a reaction pressure of 10 KPa to 1500 KPa in a solvent system; and crystallizing and vacuum-drying to obtain a bisfluorosulfonylimide salt.

In order to solve the above technical problems, the present disclosure provides a new process for a bisfluorosulfonylimide salt.

In the present disclosure, no water is generated during the reaction, and the RFSI is stable in nature and can be purified very easily, unlike HFSI and HClSI having strong acidity and strong corrosivity. Therefore, the overall reaction conditions are mild, the requirements on the equipment are low, and the equipment is not corroded, thus enabling a high-purity bisfluorosulfonylimide salt to be obtained.

The specific reaction equation of the present disclosure is as follows:

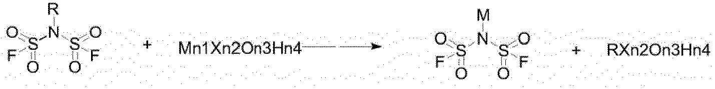

In a specific embodiment, the method for preparing a bisfluorosulfonylimide salt includes the following steps:
S1: reacting an N-alkyl substituted bisfluorosulfonylimide and a salt in a solvent system to obtain a crude product; and
S2: crystallizing and drying to obtain a bisfluorosulfonylimide salt.

The use of the N-alkyl substituted bisfluorosulfonylimide enables the system to be neutral during the reaction, so that no corrosive substances will be produced.

In a specific embodiment, a molar ratio of the N-alkyl substituted bisfluorosulfonylimide to the salt is (1: 1) to (1: 3).

In a preferred embodiment, the molar ratio of the N-alkyl-substituted bisfluorosulfonylimide to the salt is 1: 1.5.

By selecting an appropriate salt and controlling the ratio of the N-alkyl-substituted bisfluorosulfonylimide to the salt, it can be ensured that there will be fewer three wastes in the production process; moreover, there are more salts that can be chosen, which is suitable for large-scale production.

In a specific embodiment, the reaction temperature is 0°C to 150°C, and the reaction pressure is 10 KPa to 1500 KPa.

In a preferred embodiment, the reaction temperature is 10°C, and the reaction pressure is 101 KPa.

In a specific embodiment, the structural formula of the salt is Mₙ₁Xₙ₂Oₙ₃Hₙ₄; wherein n1 is a natural number ≧ 1, and each of n2, n3 and n4 is a natural number.

In a specific embodiment, the structural formula of the salt is Mₙ₁Xₙ₂Oₙ₃Hₙ₄; wherein n1 is 1, each of n2, n3 and n4 is 0, and the Mₙ₁Xₙ₂Oₙ₃Hₙ₄ is ammonia gas or single-element metal.

In a specific embodiment, the structural formula of the salt is Mₙ₁Xₙ₂Oₙ₃Hₙ₄; wherein each of n1, n2, n3 and n4 is a natural number ≧ 1; the M is selected from one of ammonium, sodium, potassium, iron, calcium, lithium, copper, zinc, cadmium, chromium, aluminum, lead, nickel, molybdenum, palladium and tin; and the X is selected from one of hydrogen, boron, carbon, silicon, nitrogen, phosphorus, arsenic, oxygen, sulfur, selenium, tellurium, fluorine, chlorine, bromine, iodine, astatine, bicarbonate, carbonate, sulfate, bisulfate, nitrate, nitrite, phosphate, hydrogen phosphate, and dihydrogen phosphate.

In a specific embodiment, the X further includes 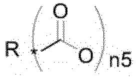 or RO, the R is -Cₙ₆Hₙ₇, wherein each of n5, n6 and n7 is a natural number ≥ 1, the R is selected from one of hydrogen, saturated aliphatic alkyl, unsaturated aliphatic alkyl, aromatic alkyl, saturated aliphatic alkyl substituted with other groups, unsaturated aliphatic alkyl substituted with other groups, aromatic alkyl substituted with other groups, and alkyl substituted with other groups; and said other groups are selected from one of formate, oxalate, hydrogen oxalate, and methoxy.

In a specific embodiment, there are no absolutely easy-to-handle raw materials. The applicant has found that, for different salts, the most suitable N-alkyl substituted bisfluorosulfonylimide and solvent will also be different. Therefore, they should be chosen according to actual needs.

In a preferred embodiment, the preparation raw materials are N-benzyl-bisfluorosulfonylimide and anhydrous lithium chloride.

In a preferred embodiment, the preparation raw materials are anhydrous cesium carbonate and N-ethyl-bisfluorosulfonylimide.

A solvent is a liquid that can dissolve gas, solid, and liquid into a homogeneous mixture. Gases and solids are conventionally called solutes, and liquids are called solvents. For a solution consisting of two liquids, the component with a higher content is usually called the solvent, and the component with a lower content is called the solute. The solvent is divided into two major categories of inorganic solvents and organic solvents. Water is the most widely used inorganic solvent, and alcohol, gasoline, chloroform, and acetone are commonly used organic solvents. Therefore, there is no distinction of optimal and worst, and a choice is made according to actual needs.

In a specific embodiment, a solvent used in the solvent system is selected from one or a combination of more than one of: diethyl carbonate, dimethyl carbonate, propylene carbonate, ethylene carbonate, ethyl methyl carbonate, methyl tert-butyl ether, tetrahydrofuran, methyl tetrahydrofuran, toluene, xylene, chlorobenzene, dichlorobenzene, trichlorobenzene, tetrachlorobenzene, methyl formate, ethyl formate, methyl acetate, ethyl acetate, propyl acetate, isopropyl acetate, n-butyl acetate, acetonitrile, ethylene glycol dimethyl ether, ethylene glycol diethyl ether, diethylene glycol dimethyl ether, acetone, diethyl ether, isopropyl ether, butyl ether, anisole, diphenyl ether, 1,4-dioxane, dichloromethane, trichloromethane, tetrachloromethane, 1,2-dichloroethane, 1,1,1-trichloroethane, 1,1,2-trichloroethane, 1,1,2,2-tetrachloroethane, 1,1,2,2-tetrachloroethylene and γ-butyrolactone.

By selecting appropriate reactants and controlling the amounts of the substances, it can be ensured that no corrosive substances will be generated during the reaction and fewer by-products will be generated during the process, thereby realizing a high purity of the product.

The present disclosure will be further described below by way of embodiments, but it is not intended to limit the present disclosure to the scope of the described embodiments. Under the premise of not violating common knowledge in the art, the above conditions can be arbitrarily combined to obtain each preferred example of the present disclosure.

For the experimental methods without specific conditions specified in the following examples, choices are made according to conventional methods and conditions, or according to product specifications.

### Examples

### Example 1

Example 1 provides a method for preparing a bisfluorosulfonylimide salt, which includes the following steps:
adding 42.3 g of anhydrous lithium chloride (dried at 105°C for 2 hours) to 800 g of dimethyl carbonate, lowering the temperature to 25°C, and adding 280 g of N-benzyl-bisfluorosulfonylimide dropwise for 2 hours; then continuing the reaction at 25°C and 101 KPa for 5 hours, followed by filtration and vacuum-concentration to a thick state; adding 400 g of toluene dropwise, stirring at 25°C for 2 hours, followed by filtering and washing with toluene; and vacuum-drying the filtered cake at 50°C to obtain lithium bisfluorosulfonylimide.

### Example 2

Example 2 provides a method for preparing a bisfluorosulfonylimide salt, which includes the following steps:
adding 26.0 g of anhydrous lithium fluoride to 400 g of n-butyl acetate, controlling the temperature at about 50°C, and adding 200 g of N-methyl-bisfluorosulfonylimide dropwise for 2 hours; then continuing the reaction at 50°C and 101 KPa for 2 hours, followed by hot filtration and vacuum-concentration to a thick state; adding 600 g of dichloromethane dropwise, stirring at 15°C for 2 hours, followed by filtering and washing with dichloromethane; and vacuum-drying the filtered cake at 60°C to obtain lithium bisfluorosulfonylimide.

### Example 3

Example 3 provides a method for preparing a bisfluorosulfonylimide salt, which includes the following steps:
adding 163.0 g of anhydrous cesium carbonate to 600 g of methyl tert-butyl ether, controlling the temperature at about 10°C, and adding 225 g of N-ethyl-bisfluorosulfonylimide dropwise for 1 hour; then continuing the reaction at 10°C and 101 KPa for 8 hours, followed by filtration and vacuum-concentration to a thick state; adding 600 g of 1,2-dichloroethane dropwise, stirring at 15°C for 2 hours, followed by filtering and washing with 1,2-dichloroethane; and vacuum-drying the filtered cake at 60°C to obtain cesium bisfluorosulfonylimide.

### Example 4

Example 4 provides a method for preparing a bisfluorosulfonylimide salt, which includes the following steps:
adding 56.0 g of anhydrous potassium hydroxide to 300 g of acetonitrile, controlling the temperature at about 25°C, and adding 230 g of N-propenyl-bisfluorosulfonylimide dropwise for 2 hours; then continuing the reaction at 25°C and 101 KPa for 4 hours, followed by filtration and vacuum-concentration to a thick state; adding 600 g of o-xylene dropwise, stirring at 15°C for 2 hours, followed by filtering and washing with o-xylene; and vacuum-drying the filtered cake at 60°C to obtain potassium bisfluorosulfonylimide.

### Example 5

Example 5 provides a method for preparing a bisfluorosulfonylimide salt, which includes the following steps:
adding 68.1 g of sodium ethoxide to 300 g of tetrahydrofuran, controlling the temperature at about 35°C, and adding 235 g of N-propyl-bisfluorosulfonylimide dropwise for 2 hours; then continuing the reaction at 35°C and 101 KPa for 3 hours, followed by filtration and vacuum-concentration to a thick state; adding 600 g of dichloromethane dropwise, stirring at 15°C for 2 hours, followed by filtering and washing with dichloromethane; and vacuum-drying the filtered cake at 60°C to obtain sodium bisfluorosulfonylimide.

### Comparative Example 1

Comparative Example 1 provides a method for preparing a bisfluorosulfonylimide salt, which includes the following steps:
adding 163.0 g of anhydrous cesium carbonate to 600 g of methyl tert-butyl ether, controlling the temperature at about 10°C, and adding 225 g of bisfluorosulfonylimide dropwise for 1 hour; then continuing the reaction at 10°C and 150 KPa for 8 hours, followed by filtration and vacuum-concentration to a thick state; adding 600 g of 1,2-dichloroethane dropwise, stirring at 15°C for 2 hours, followed by filtering and washing with 1,2-dichloroethane; and vacuum-drying the filtered cake at 60°C to obtain cesium bisfluorosulfonylimide.

### Comparative Example 2

Comparative Example 2 provides a method for preparing a bisfluorosulfonylimide salt, which includes the following steps:
adding 163.0 g of anhydrous cesium carbonate to 600 g of methyl tert-butyl ether, controlling the temperature at about 10°C, and adding 225 g of N-ethyl-bisfluorosulfonylimide dropwise for 1 hour; then continuing the reaction at 200°C and 250 KPa for 8 hours, followed by filtration and vacuum-concentration to a thick state; adding 600 g of 1,2-dichloroethane dropwise, stirring at 15°C for 2 hours, followed by filtering and washing with 1,2-dichloroethane; and vacuum-drying the filtered cake at 60°C to obtain cesium bisfluorosulfonylimide.

### Comparative Example 3

Comparative Example 3 provides a method for preparing a bisfluorosulfonylimide salt, which includes the following steps:
adding 163.0 g of anhydrous cesium carbonate to 600 g of methyl tert-butyl ether, controlling the temperature at about 10°C, and adding 225 g of bisfluorosulfonylimide dropwise for 1 hour; then continuing the reaction at 250°C and 200 KPa for 8 hours, followed by filtration and vacuum-concentration to a thick state; adding 600 g of 1,2-dichloroethane dropwise, stirring at 15°C for 2 hours, followed by filtering and washing with 1,2-dichloroethane; and vacuum-drying the filtered cake at 60°C to obtain cesium bisfluorosulfonylimide.

### Evaluation and test on performance

The yield, moisture, acid value and product melting point of the bisfluorosulfonylimide salts prepared in examples 1 to 5 are tested below, and the results of test are shown in table 1.

**Table 1**

| | Product | Mass of product | Yield | Moisture | Acid value | Melting point |
|---|---|---|---|---|---|---|
| Example 1 | lithium bisfluorosulfonylimi de | 175.1 g | 93.5% | < 100ppm | < 100ppm | 124°C~1 26°C |
| Example 2 | lithium bisfluorosulfonylimi de | 170 g | 90.7% | < 100ppm | < 100ppm | 124°C~1 26°C |
| Example 3 | cesium bisfluorosulfonylimi de | 300 g | 95.8% | < 100ppm | < 100ppm | 112°C~1 14°C |
| Example 4 | potassium bisfluorosulfonylimi de | 179.6 g | 82.0% | < 100ppm | < 100ppm | 94°C~96 °C |
| Example 5 | sodium bisfluorosulfonylimi de | 182.2 g | 89.7% | < 100ppm | < 100ppm | 115°C~1 17°C |
| Comparative Example 1 | cesium bisfluorosulfonylimi de | 203.9 g | 65.1% | < 100ppm | < 100ppm | 112°C~1 14°C |
| Comparative Example 2 | cesium bisfluorosulfonylimi de | 226.4 g | 72.3% | < 100ppm | < 100ppm | 112°C~1 14°C |
| Comparative Example 3 | cesium bisfluorosulfonylimi de | 152.2 g | 48.6% | < 100ppm | < 100ppm | 112°C~1 14°C |

## Claims

1. A method for preparing a bisfluorosulfonylimide salt, comprising the following steps:
S1: reacting an N-alkyl substituted bisfluorosulfonylimide and a salt in a solvent system to obtain a crude product; and
S2: crystallizing and drying to obtain a bisfluorosulfonylimide salt,
and wherein the structural formula of the salt is Mₙ₁Xₙ₂Oₙ₃Hₙ₄; and wherein each of n1, n2, n3 and n4 is a natural number ≧ 1; the M is selected from one of ammonium, sodium, potassium, iron, calcium, lithium, copper, zinc, cadmium, chromium, aluminum, lead, nickel, molybdenum, palladium and tin; and the X is selected from one of hydrogen, boron, carbon, silicon, nitrogen, phosphorus, arsenic, oxygen, sulfur, selenium, tellurium, fluorine, chlorine, bromine, iodine, astatine, bicarbonate, carbonate, sulfate, bisulfate, nitrate, nitrite, phosphate, hydrogen phosphate, and dihydrogen phosphate and
wherein a molar ratio of the N-alkyl substituted bisfluorosulfonylimide to the salt is (1: 1) to (1: 3) and
wherein the reaction temperature is 0°C to 150°C, and the reaction pressure is 10 KPa to 1500 KPa.

2. The preparing method according to claim 1, wherein the structural formula of the N-alkyl substituted bisfluorosulfonylimide is: 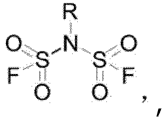 R is -Cₙ₈Hₙ₉, and each of n8 and n9 is a natural number ≥ 1.

3. The preparing method according to claim 1, wherein a solvent used in the solvent system is selected from one or a combination of more than one of: diethyl carbonate, dimethyl carbonate, propylene carbonate, ethylene carbonate, ethyl methyl carbonate, methyl tert-butyl ether, tetrahydrofuran, methyl tetrahydrofuran, toluene, xylene, chlorobenzene, dichlorobenzene, trichlorobenzene, tetrachlorobenzene, methyl formate, ethyl formate, methyl acetate, ethyl acetate, propyl acetate, isopropyl acetate, n-butyl acetate, acetonitrile, ethylene glycol dimethyl ether, ethylene glycol diethyl ether, diethylene glycol dimethyl ether, acetone, diethyl ether, isopropyl ether, butyl ether, anisole, diphenyl ether, 1,4-dioxane, dichloromethane, trichloromethane, tetrachloromethane, 1,2-dichloroethane, 1,1,1-trichloroethane, 1,1,2-trichloroethane, 1,1,2,2-tetrachloroethane, 1,1,2,2-tetrachloroethylene and γ-butyrolactone.

## Patentansprüche

1. Verfahren zum Herstellen eines Bisfluorsulfonylimidsalzes, umfassend die folgenden Schritte:
S1: Reagieren eines N-alkylsubstituierten Bisfluorsulfonylimids und eines Salzes in einem Lösungsmittelsystem, um ein Rohprodukt zu erhalten; und
S2: Kristallisieren und Trocknen, um ein Bisfluorsulfonylimidsalz zu erhalten,
und wobei die Strukturformel des Salzes Mₙ₁Xₙ₂Oₙ₃Hₙ₄ ist; und wobei jedes von n1, n2, n3 und n4 eine natürliche Zahl ≥ 1 ist; das M ausgewählt ist aus einem von Ammonium, Natrium, Kalium, Eisen, Kalzium, Lithium, Kupfer, Zink, Kadmium, Chrom, Aluminium, Blei, Nickel, Molybdän, Palladium und Zinn; und das X ausgewählt ist aus einem von Wasserstoff, Bor, Kohlenstoff, Silizium, Stickstoff, Phosphor, Arsen, Sauerstoff, Schwefel, Selen, Tellur, Fluor, Chlor, Brom, Jod, Astatin, Bikarbonat, Karbonat, Sulfat, Bisulfat, Nitrat, Nitrit, Phosphat, Wasserstoffphosphat und Diwasserstoffphosphat und
wobei ein Molverhältnis des N-alkylsubstituierten Bisfluorsulfonylimids zu dem Salz (1:1) bis (1:3) ist und
wobei die Reaktionstemperatur 0°C bis 150°C ist und der Reaktionsdruck 10 KPa bis 1500 KPa ist.

2. Herstellungsverfahren nach Anspruch 1, wobei die Strukturformel des N-alkylsubstituierten Bisfluorsulfonylimids ist: 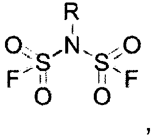 R -Cₙ₈Hₙ₉ ist und jedes von n8 und n9 eine natürliche Zahl ≥ 1 ist.

3. Herstellungsverfahren nach Anspruch 1, wobei ein Lösungsmittel, das in dem Lösungsmittelsystem verwendet wird, ausgewählt ist aus einem oder einer Kombination von mehr als einem von: Diethylkarbonat, Dimethylkarbonat, Propylenkarbonat, Ethylenkarbonat, Ethylmethylkarbonat, Methyl-tert-Butylether, Tetrahydrofuran, Methyltetrahydrofuran, Toluol, Xylol, Chlorbenzol, Dichlorbenzol, Trichlorbenzol, Tetrachlorbenzol, Methylformiat, Ethylformiat, Methylazetat, Ethylazetat, Propylazetat, Isopropylazetat, n-Butylazetat, Azetonitril, Ethylenglykoldimethylether, Ethylenglykoldiethylether, Diethylenglykoldimethylether, Azeton, Diethylether, Isopropylether, Butylether, Anisol, Diphenylether, 1,4-Dioxan, Dichlormethan, Trichlormethan, Tetrachlormethan, 1,2-Dichlorethan, 1,1,1-Trichlorethan, 1,1,2-Trichlorethan, 1,1,2,2-Tetrachlorethan, 1,1,2,2-Tetrachlorethylen und γ-Butyrolacton.

## Revendications

1. Procédé pour préparer un sel de bisfluorosulfonylimide, comprenant les étapes suivantes :
S1 : faire réagir un bisfluorosulfonylimide substitué par un N-alkyle et un sel dans un système de solvant pour obtenir un produit brut ; et
S2 : cristalliser et sécher pour obtenir un sel de bisfluorosulfonylimide,
et dans lequel la formule structurale du sel est Mₙ₁Xₙ₂Oₙ₃Hₙ₄ ; et dans lequel chacun de n1, n2, n3 et n4 est un nombre naturel ≥ 1 ; le M est choisi parmi l'un parmi l'ammonium, le sodium, le potassium, le fer, le calcium, le lithium, le cuivre, le zinc, le cadmium, le chrome, l'aluminium, le plomb, le nickel, le molybdène, le palladium et l'étain ; et le X est choisi parmi l'un parmi l'hydrogène, le bore, le carbone, le silicium, le nitrogène, le phosphore, l'arsenic, l'oxygène, le soufre, le sélénium, le tellure, le fluor, le chlore, le brome, l'iode, l'astatine, le bicarbonate, le carbonate, le sulfate, le bisulfate, le nitrate, le nitrite, le phosphate, le phosphate d'hydrogène et le phosphate de dihydrogène et
dans lequel un rapport molaire du bisfluorosulfonylimide substitué par un N-alkyle au sel est de (1:1) à (1:3) et
dans lequel la température de réaction est de 0 °C à 150 °C, et la pression de réaction est de 10 KPa à 1500 KPa.

2. Procédé de préparation selon la revendication 1, dans lequel la formule structurale du bisfluorosulfonylimide substitué par un N-alkyle est : 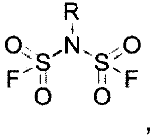 R est -Cₙ₈Hₙ₉ , et chacun de n8 et n9 est un nombre naturel ≥ 1.

3. Procédé de préparation selon la revendication 1, dans lequel un solvant utilisé dans le système de solvant est choisi parmi l'un ou une combinaison de plus d'un parmi : le carbonate de diéthyle, le carbonate de diméthyle, le carbonate de propylène, le carbonate d'éthylène, le carbonate d'éthyle et de méthyle, l'éther tert-butylique de méthyle, le tétrahydrofurane, le méthyltétrahydrofurane, le toluène, le xylène, le chlorobenzène, le dichlorobenzène, le trichlorobenzène, le tétrachlorobenzène, le formiate de méthyle, le formiate d'éthyle, l'acétate de méthyle, l'acétate d'éthyle, l'acétate de propyle, l'acétate d'isopropyle, l'acétate de n-butyle, l'acétonitrile, l'éther diméthylique d'éthylène glycol, l'éther diéthylique d'éthylène glycol, l'éther diméthylique de diéthylène glycol, l'acétone, l'éther diéthylique, l'éther isopropylique, l'éther butylique, l'anisole, l'éther diphénylique, le 1,4-dioxane, le dichlorométhane, le trichlorométhane, le tétrachlorométhane, le 1,2-dichloroéthane, le 1,1,1-trichloroéthane, le 1,1,2-trichloroéthane, le 1,1,2,2-tétra-chloroéthane, le 1,1,2,2-tétrachloroéthylène et la γ-butyrolactone.
